# EUROPEAN PATENT SPECIFICATION

(11) **EP 3 808 758 B1**
(45) Date of publication and mention of the grant of the patent: **25.10.2023**
(21) Application number: 19820450.5
(22) Date of filing: 28.05.2019
(51) Int. Cl.: C07K 1/02, C07K 1/10, C07K 5/078, C07D 277/56

(54) **METHOD FOR PRODUCING AMIDE**
VERFAHREN ZUR HERSTELLUNG VON AMID
PROCÉDÉ DE PRODUCTION D'AMIDE

(30) Priority: 15.06.2018 JP 2018114577
(43) Date of publication of application: 21.04.2021
(73) Proprietor: Yokogawa Electric Corporation, Tokyo 180-8750 (JP)
(72) Inventor: FUSE, Shinichiro, Tokyo 152-8550 (JP); NAKAMURA, Hiroyuki, Tokyo 152-8550 (JP); OTAKE, Yuma, Tokyo 152-8550 (JP); OGAWA, Jun-ichi, Musashino-shi, Tokyo 180-8750 (JP); MIYAZAKI, Shun-ichi, Musashino-shi, Tokyo 180-8750 (JP); ITO, Atsushi, Musashino-shi, Tokyo 180-8750 (JP)
(74) Representative: Winter, Brandl - Partnerschaft mbB
(86) International application number: PCT/JP2019/021106
(87) International publication number: WO 2019/239879

(56) References cited:
- JP-A- H08 504 779
- JP-A- 2006 502 128
- US-B1- 6 489 479
- SHINICHIRO FUSE ET AL: "Total synthesis of feglymycin based on a linear/convergent hybrid approach using micro-flow amide bond formation", NATURE COMMUNICATIONS, VOL. 10, vol. 7, 28 November 2016 (2016-11-28), pages 1-7, XP055662919, DOI: 10.1038/ncomms13491 Retrieved from the Internet: URL:https://www.nature.com/articles/ncomms 13491.pdf>
- CHRISTIAN A.G.N. MONTALBETTI ET AL: "Amide bond formation and peptide coupling", TETRAHEDRON, vol. 61, no. 46, 1 November 2005 (2005-11-01), pages 10827-10852, XP055535483, AMSTERDAM, NL ISSN: 0040-4020, DOI: 10.1016/j.tet.2005.08.031
- FUSE, S. et al.: "Total synthesis of feglymycin based on a linear/convergent hybrid approach using micro-flow amide bond formation", Nature communications, vol. 10, no. 1038, 2016, pages 1-7, XP055662919,

## Description

### [Technical Field]

The present invention relates to a method for producing an amide.

### [Background Art]

In peptide synthesis, a carboxylic group of an amino acid is activated and reacted with an amino group of the amino acid, a coupling reaction is caused to form amide bonds, these operations are repeated, and thus the amino acid is sequentially extended. Several methods are known as methods of activating carboxylic groups. There are a method of synthesizing peptides while minimizing isomerization and production of byproducts using a condensing agent having a low degree of activation and a method of synthesizing peptides using an activation agent in a short time.

Examples of a method of activating the carboxylic group using a highly active activation agent include acid chloride methods and acid anhydride methods. Compared with an activation method using a condensing agent having a low degree of activation, these acid chloride methods and acid anhydride methods have advantages such as low unit price, a small amount of produced byproducts derived from an activation agent, and the like because the structure of the activation agent is simpler.

The acid anhydride methods are classified into a symmetric acid anhydride method and a mixed acid anhydride method.

For example, in Non Patent Literature 1 to 2, a method of synthesizing an amide using a symmetric acid anhydride as an active species of a carboxylic acid is disclosed.

The symmetric acid anhydride method disclosed in Non Patent Literature 1 to 2 can be said to be a method including a first step in which a symmetric acid anhydride is produced by a condensation reaction between carboxylic acids and a second step in which a coupling reaction between the symmetric acid anhydride and an amine is performed.

In addition, for example, Non Patent Literature 3 discloses a method of synthesizing an amide using a mixed acid anhydride as an active species of a carboxylic acid.

It is described in Non Patent Literature 3 that a carboxylic acid and isopropyl chloroformate are mixed with a first micro mixer, a mixed acid anhydride is synthesized in a short time, and subsequently, a solution containing the mixed acid anhydride, an amine and a catalyst (base) are immediately mixed with a second micro mixer to perform amidation so that the synthesized mixed acid anhydride is not racemized.

The mixed acid anhydride method disclosed in Non Patent Literature 3 can be said to be a method including a first step in which a carboxylic acid reacts with chloroformate to obtain a mixed acid anhydride, a second step in which a base is added to the mixed acid anhydride to obtain an acylpyridinium species, and a third step in which a coupling reaction between the acylpyridinium species and an amine is performed to obtain an amide.

### [Citation List]

### [Non Patent Literature]

[Non Patent Literature 1]
   "Efficient Amide Bond Formation through a Rapid and StrongActivation of Carboxylic Acids in a Microflow Reactor," Fuse, S. Mifune, Y. Takahashi, T., Angew Chem. Int. Ed. 53, 851-855 (2014).
[Non Patent Literature 2]
   "Total synthesis of feglymycin based on a linear/convergent hybrid approach using micro-flow amide bond formation", Fuse S., Mifune Y., Nakamura H., Tanaka H., Nat. Commun. 7, vol. 10, p. 1-7, 13491 (2016).
[Non Patent Literature 3]
   Yuma Otake, Hiroyuki Nakamura, Shinichiro Fuse, "An efficient synthesis of N-methylated peptide using micro-flow methodology," March 16, 2017, The 97th Annual Meeting of the Chemical Society of Japan, 3F4-14

### [Summary of Invention]

### [Technical Problem]

However, in the symmetric acid anhydride method, since the reactivity of the symmetric acid anhydride with respect to an amine is weak, there is a problem that a coupling reaction with an amine having low nucleophilicity takes a long time or the reaction does not proceed.

In addition, the mixed acid anhydride method has a problem that an ester, which is an undesired compound, is produced due to a reaction between an acylpyridinium species and counter anions for an acylpyridinium species.

The present invention has been made in order to address the above problems, and an object of the present invention is to provide a method for producing an amide in which, in the reaction in which carboxylic groups are activated and reacted with an amino group, a coupling reaction is caused to form amide bonds, the reaction efficiency is favorable and side reactions are unlikely to occur.

### [Solution to Problem]

The present invention provides a method for producing an amide as defined in the claims.

### [Advantageous Effects of Invention]

According to the present invention, it is possible to provide a method for producing an amide which has favorable reaction efficiency and is unlikely to cause a side reaction.

### [Brief Description of Drawings]

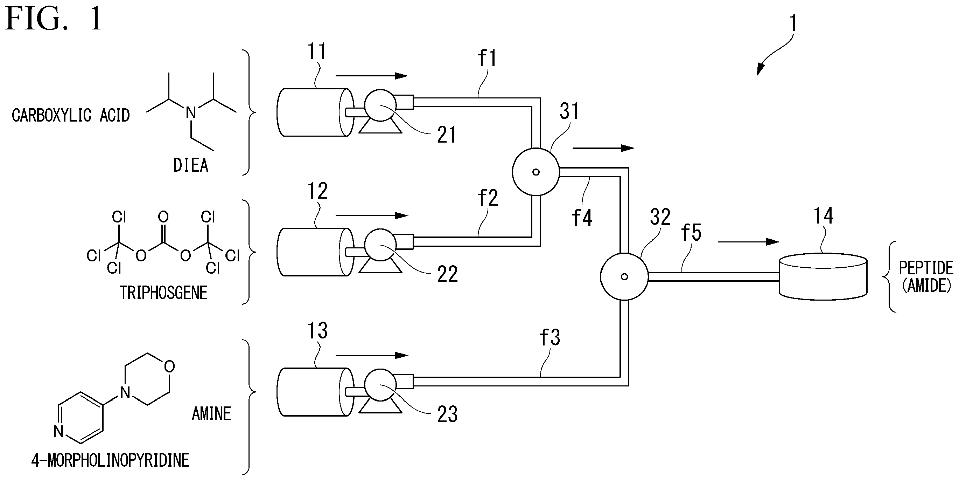

Fig. 1 is a schematic view showing a schematic configuration of a distribution system reaction device 1.

### [Description of Embodiments]

A method for producing an amide according to an embodiment of the present invention will be described below.

### «Method for producing amide»

The method for producing an amide according to the present invention comprises dehydrating and condensing carboxylic acids; and reacting the dehydrated and condensed carboxylic acids with any one or more base, which is then reacted with an amine simultaneously.

The base according to the present invention is selected from the group consisting of pyridine and pyridine derivatives to obtain an acylpyridinium cation.

Moreover, the carboxylic acids are amino acids or amino acid derivatives and the amine is an amino acid or an amino acid derivative.

Here, the base is one that produces a cationically active species.

The production method includes the following Processes 1 to 3.

Process 1: a process in which carboxylic acids are dehydrated and condensed to obtain an acid anhydride.

Process 2: a process in which the acid anhydride obtained in Process 1 is reacted with a base to obtain a cationically active species.

Process 3: a process in which the cationically active species obtained in Process 2 is reacted with an amine to produce an amide.

The above processes will be described below. Here, the reaction of the method for producing an amide according to the present invention is not limited to reactions exemplified in the following processes.

### <Process 1>

Process 1 is a process in which carboxylic acids are dehydrated and condensed to obtain an acid anhydride.

The carboxylic acids are amino acids or amino acids derivatives having a carboxylic group at the end of the molecule and may be represented by the following General Formula (1). 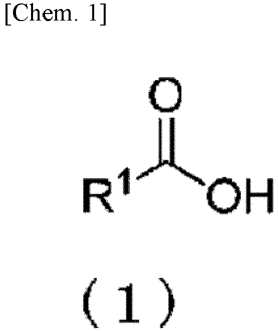 (in the formula, R¹ represents CH(R⁰)NH₂, wherein R⁰ represents a side chain of an amino acid or amino acid derivative)

The carboxylic acids, which are amino acids or amino acid derivatives, may be deprotonated into carboxylate ions and may be represented by the following General Formula (1i). 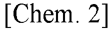 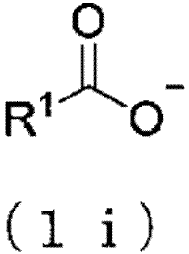 (in the formula, R¹ represents CH(R⁰)NH₂, wherein R⁰ represents a side chain of an amino acid or amino acid derivative)

Deprotonation of the carboxylic acids can be achieved, for example, by placing the carboxylic acids in the presence of a base having low nucleophilicity such as N,N-diisopropylethylamine (DIEA) in the reaction system.

The presence of a base means, for example, in a solvent in which a base is added. The type of the base is not particularly limited as long as it allows the carboxylic acid, which is an amino acid or amino acid derivative, to be deprotonated in the reaction system.

In Process 1 in the method for producing an amide according to the embodiment, carboxylic acids represented by the following General Formula (1) and carboxylic acids represented by the following General Formula (1)' are dehydrated and condensed to obtain an acid anhydride represented by the following General Formula (2). The acid anhydride can be obtained, for example, by reacting the carboxylic acids with a phosgene or a phosgene equivalent that decomposes in a reaction system and produces a phosgene. 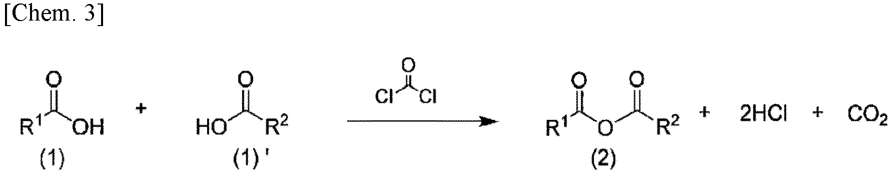 (in the formula, R¹ and R² each independently represent CH(R⁰)NH₂, wherein R⁰ independently represents a side chain of an amino acid or amino acid derivative)

The phosgene equivalent is one that decomposes in a reaction system and produces a phosgene, and can be used in substantially the same way as a phosgene in a synthetic reaction. Examples of phosgene equivalents include diphosgene and triphosgene.

In the dehydration condensation, carboxylic acids of different types may be dehydrated and condensed, and carboxylic acids of the same type may be dehydrated and condensed. That is, in Formulae (1) and (1)', R¹ and R² may be the same as or different from each other.

When R¹ and R² are the same, the acid anhydride represented by General Formula (2) is a symmetric acid anhydride. When R¹ and R² are the same, counter anions of a cationically active species produced in Process 2 to be described below are the same as carboxylate ions before activation. Counter anions may self-react with a cationically active species, but if counter anions are the same as carboxylate ions before activation, even if self-reacted, the product becomes the same as a symmetric acid anhydride before it is activated into a cationically active species.

Therefore, when R¹ and R² are the same, there are advantages that the type of amide obtained in the reaction system is uniform and it is easy to systematically obtain a desired type of the reaction product.

As already stated above, the carboxylic acid is an amino acid or an amino acid derivative. The carboxylic acid here includes a carboxylic acid that is a precursor of an acid anhydride. Regarding the amino acid, the amino acid is preferably an α-amino acid. In addition, generally, since amino acids constituting peptides or proteins in a living body are of an L-type, the amino acids are preferably an L-type. The α-amino acid may be a compound represented by the following General Formula (1-1). 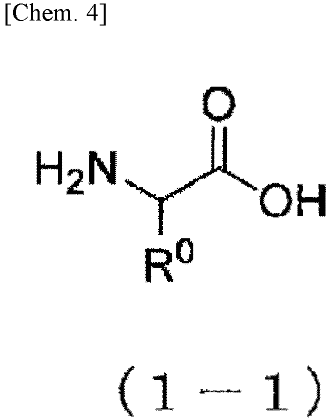 (in the formula, R⁰ represents a side chain of an amino acid)

The amino acids may be 20 types of amino acids which constitute peptides or proteins in a living body and are encoded as genetic information. These amino acids include alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine. In addition, the amino acid may be a type of amino acid that is not encoded as genetic information such as cysteine.

For example, R⁰ in Formula (1-1) is "-CH₃" when the amino acid is alanine, "-H" when the amino acid is glycine, "-CH(CH₃)₂" when the amino acid is valine, and "-CH(CH₃)CH₂CH₃" when the amino acid is isoleucine. The same also applies to other amino acids.

The amino acid need not be an α-amino acid. For example, it may be a β-amino acid such as β-alanine.

The carboxylic acid may be an amino acid derivative. The amino acid derivative may be a compound having substantially the same properties as the amino acid, and may be a natural type that occurs naturally or a type which has modifications such as alternation, addition, or substitution of a functional group different from those of the natural type.

As an example of a case having substantially the same properties as an amino acid, a case in which amino acid derivatives can be incorporated into an enzyme that uses an amino acid as a substrate and a case in which amino acid derivatives can be bound to molecules that bind to an amino acid may be exemplified.

Examples of amino acid derivatives include those in which one or more hydrogen atoms or groups in the amino acid are substituted with other groups (substituents). As an example of amino acid derivatives, a protected amino acid in which a functional group is protected by a protecting group may be exemplified. The protecting group has a function of inactivating a reactive functional group. It is possible to deprotect the protecting group and return the protected functional group to its unprotected state. Here, the fact that the functional group is protected means that atoms constituting the functional group are substituted with a protecting group. Examples of sites protected by a protecting group include any one or more sites selected from the group consisting of amino groups, carboxylic groups, and side chains. One or two or more functional groups contained in the side chain may be protected by a protecting group. In Process 1, it is preferable that amino groups and/or functional groups in the side chain be protected so that the reaction of the reactive functional group other than carboxylic groups is prevented.

The type of the protecting group is not particularly limited, and can be appropriately selected depending on the type of the functional group to be protected. Examples of amino group protecting groups include carbamate-based, sulfonamide-based, acyl-based, and alkyl-based protecting groups, and the present invention is not limited thereto.

Examples of carbamate-based protecting groups include 2-benzyloxycarbonyl groups (sometimes abbreviated as -Z or -Cbz), tert-butyloxycarbonyl groups (sometimes abbreviated as -Boc), allyloxycarbonyl groups (sometimes abbreviated as -Alloc), 2,2,2-trichloroethoxycarbonyl groups (sometimes abbreviated as -Troc), 2-(trimethylsilyl)ethoxycarbonyl groups (sometimes abbreviated as -Teoc), 9-fluorenylmethyloxycarbonyl groups (sometimes abbreviated as -Fmoc), p-nitrobenzyloxycarbonyl groups (sometimes abbreviated as -Z(NOz)), and p-biphenylisopropyloxycarbonyl groups (sometimes abbreviated as -Bpoc).

Examples of sulfonamide-based protecting groups include p-toluenesulfonyl groups (sometimes abbreviated as -Ts or -Tos), 2-nitrobenzene sulfonyl groups (sometimes abbreviated as -Ns), 2,2,4,6,7-pentamethyldihydrobenzofuran-5-sulfonyl (sometimes abbreviated as -Pbf), 2,2,5,7,8-pentamethylchroman-6-sulfonyl (sometimes abbreviated as -Pmc), and 1,2-dimethylindole-3-sulfonyl (sometimes abbreviated as - MIS).

### <Process 2>

Process 2 is a process in which the acid anhydride obtained in Process 1 is reacted with a base to obtain a cationically active species.

In Process 2 in the method for producing an amide according to the embodiment, an acid anhydride represented by the following General Formula (2) is reacted with a base represented by B to obtain a cationically active species represented by the following General Formula (4). Here, in the reaction, a compound represented by the following General Formula (5) is produced as a counter anion of a cationically active species. 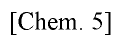 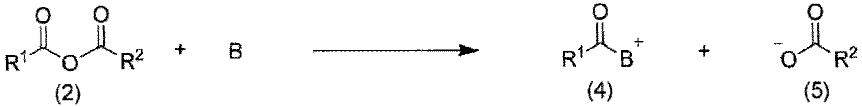 (in the formula, R¹ and R² each independently represent CH(R⁰)NH₂, wherein R⁰ independently represents a side chain of an amino acid or amino acid derivative)

The base in Process 2 reacts with the acid anhydride to produce a cationically active species, and the base may preferably be a base having high nucleophilicity. Moreover, the base is any one or more selected from the group consisting of pyridine and pyridine derivatives.

The pyridine derivative may be one in which one or more hydrogen atoms of pyridine are substituted with other groups and is not particularly limited as long as it has properties of a base, and the pyridine and pyridine derivative are preferably a compound represented by the following General Formula (3-1). 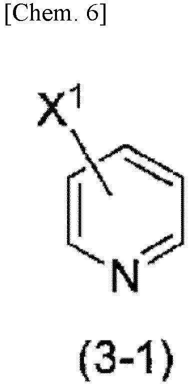 (in the formula, X¹ represents a hydrogen atom or any group selected from among the groups represented by the following Formulae (a) to (c)) 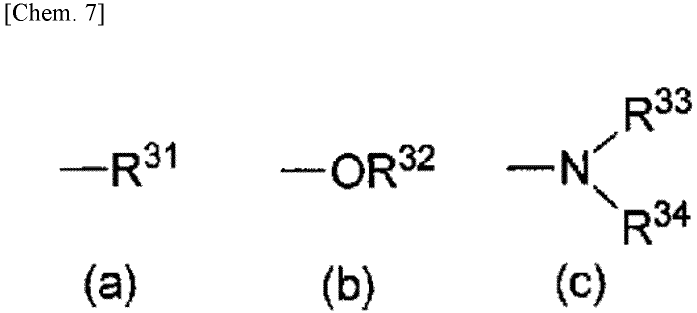 (in the formula, R³¹, R³², R³³ and R³⁴ each independently represent an alkyl group; R³³ and R³⁴ may be bonded to each other to form a ring, and one methylene group that is not directly bonded to R³³ or R³⁴ in the alkyl group may be substituted with an oxygen atom)

The alkyl group for R³¹, R³², R³³ and R³⁴ may be linear, branched or cyclic. The cyclic alkyl group may be either monocyclic or polycyclic. The alkyl group may have 1 to 20 carbon atoms, 1 to 15 carbon atoms, or 1 to 10 carbon atoms.

Examples of linear or branched alkyl groups include methyl groups, ethyl groups, n-propyl groups, isopropyl groups, n-butyl groups, isobutyl groups, sec-butyl groups, tert-butyl groups, n-pentyl groups, isopentyl groups, neopentyl groups, tert-pentyl groups, 1-methylbutyl groups, n-hexyl groups, 2-methylpentyl groups, 3-methylpentyl groups, 2,2-dimethylbutyl groups, 2,3-dimethylbutyl groups, n-heptyl groups, 2-methylhexyl groups, 3-methylhexyl groups, 2,2-dimethylpentyl groups, 2,3-dimethylpentyl groups, 2,4-dimethylpentyl groups, 3,3-dimethylpentyl groups, 3-ethylpentyl groups, 2,2,3-trimethylbutyl groups, n-octyl groups, isooctyl groups, nonyl groups, decyl groups, undecylic groups, dodecyl groups, tridecylic groups, tetradecyl groups, pentadecyl groups, hexadecyl groups, heptadecyl groups, octadecyl groups, nonadecyl groups, and icosyl groups.

The compound represented by General Formula (3-1) is preferably a compound represented by the following General Formula (3-1-1). When X¹ is any group selected from among the groups represented by Formulae (a) to (c) other than a hydrogen atom, X¹ effectively functions as an electron donating group according to bonding to a relevant position, and the nucleophilicity of N atoms of a pyridine ring tends to become better. 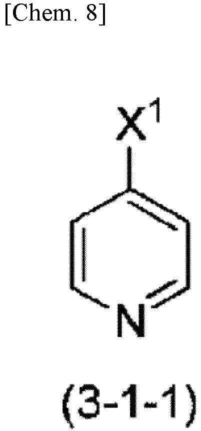 (in Formula (3-1-1), X¹ has the same meaning as X¹ in Formula (3-1))

In the compound represented by General Formula (3-1), X¹ is a group represented by Formula (c), R³³ and R³⁴ are bonded to each other to form a ring, and regarding a case in which one methylene group that is not directly bonded to R³³ or R³⁴ in the alkyl group is substituted with an oxygen atom, 4-morpholinopyridine represented by the following Formula (3-1-2) is included.

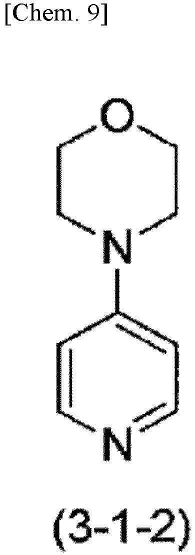

Preferable examples of pyridine and pyridine derivatives include pyridine, the above 4-morpholinopyridine, N,N-dimethyl-4-aminopyridine, 4-pyrrolidinopyridine and 4-methoxypyridine. Among these, 4-morpholinopyridine and N,N-dimethyl-4-aminopyridine are particularly preferably used because an amide synthesis yield per unit time is high and it is possible to significantly reduce the amount of side-reaction products produced.

Using pyridine and pyridine derivatives, the cationically active species is an acylpyridinium cation (an acylpyridinium species). The acylpyridinium species has high electrophilicity. Therefore, even the reaction with an amine having low nucleophilicity to be described below can proceed at a very high rate, and it is possible to significantly reduce the amount of side-reaction products produced.

### <Process 3>

Process 3 is a process in which the cationically active species obtained in Process 2 is reacted with an amine to produce an amide.

In Process 3 in the method for producing an amide according to the embodiment, a cationically active species represented by the following General Formula (4) and an amine represented by the following General Formula (6) are reacted to obtain an amide represented by the following General Formula (7). 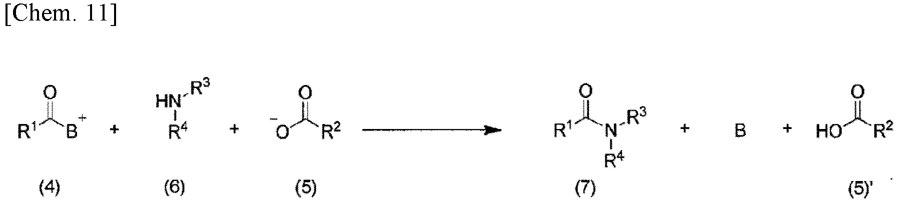 (in the formula, R¹ and R² each independently represent CH(R⁰)NH₂, wherein R⁰ independently represents a side chain of an amino acid or amino acid derivative)

The amine is an amino acid or an amino acid derivative.

Examples of amino acids and amino acid derivatives include those exemplified as the carboxylic acid.

Therefore, Formula (6) represents an amino acid, -R³ and -R⁴ may be, for example, -H and -CH(R⁰)COOH.

As an example of amino acid derivatives, a protected amino acid in which a functional group is protected by a protecting group may be exemplified. Examples of sites protected by a protecting group include any one or more sites selected from the group consisting of amino groups, carboxylic groups, and side chains. One or two or more functional groups contained in the side chain may be protected by a protecting group. In Process 3, it is preferable that carboxylic groups and/or functional groups in the side chain be protected so that the reaction of the reactive functional group other than amino groups is prevented.

The type of the protecting group is not particularly limited, and can be appropriately selected depending on the type of the functional group to be protected. Carboxylic groups may be protected by simply being neutralized in the form of salts, but are generally protected in the form of esters. Examples of esters include benzyl esters (sometimes abbreviated as Bn or BZl) in addition to alkyl esters such as methyl and ethyl, and the present invention is not limited thereto.

In the method for producing an amide according to the embodiment, the cationically active species is reacted with an amine in Process 3. Here, the method for producing an amide according to the embodiment has an advantage that the reaction rate does not depend on the nucleophilicity of the amine due to high electrophilicity of the cationically active species.

Therefore, the method for producing an amide according to the embodiment is suitable for the reaction with an amine having low nucleophilicity. Specific examples of amines having low nucleophilicity may include amines having a nucleophilicity lower than those of 18 amino acids excluding valine and isoleucine from 20 amino acids which constitute proteins and are encoded as genetic information, and more specific examples thereof can include valine, isoleucine, an N-alkylated amino acid, and derivatives thereof. The N-alkylated amino acid may be one in which one or two hydrogen atoms of an amino group bonded to α carbon are substituted with an alkyl group and is preferably N-methyl amino acid in which one hydrogen atom is substituted with a methyl group. In the related art, these amines having low nucleophilicity are difficult to use for synthesis in an acid anhydride method. However, according to the method for producing an amide of the embodiment, it is possible to use amines having low nucleophilicity which have been difficult to use for synthesis in an acid anhydride method in the related art, and in this respect as well, the method for producing an amide according to the embodiment is revolutionary.

For example, an acid anhydride method is performed under conditions shown in Example 1, the acid anhydride produced in Example 1 is reacted with an amine whose nucleophilicity is desired to be determined, and the nucleophilicity of the amine here can be determined from the degree of reaction efficiency.

In the present embodiment, the amount of each compound used during the reactions in Processes 1 to 3 may be appropriately adjusted according to a desired reaction in consideration of the types of these compounds.

A molar equivalent ratio (activated carboxylic acid:amine) between the activated carboxylic acid and the amine in the reaction system may be 10:1 to 1/10:1, 5:1 to 1/5:1 or 3:1 to 1/3:1. The activated carboxylic acid is, for example, the compound represented by General Formula (4). According to the method for producing an amide of the embodiment, even if a relatively small amount of an amine, which is close to an equivalent amount, is reacted with the activated carboxylic acid, it is possible to produce an amide with high efficiency.

In the present embodiment, the reaction time for each process may be appropriately adjusted according to other conditions such as the reaction temperature. As an example, the reaction time in Process 1 may be 0.05 seconds to 30 minutes, 0.1 seconds to 5 minutes, or 0.5 seconds to 30 seconds. When Process 2 and Process 3 are performed simultaneously, the reaction time for Process 2 and Process 3 may be 1 second to 60 minutes, 5 seconds to 30 minutes, or 1 minute to 10 minutes.

In the present embodiment, the temperature (reaction temperature) during the reactions in Processes 1 to 3 may be appropriately adjusted according to the type of compounds used in Processes 1 to 3. As an example, the reaction temperature is preferably in a range of 0 to 100 °C and more preferably in a range of 20 to 50 °C.

In the present embodiment, the reactions in Process 1 to Process 3 may be performed in the coexistence of a solvent. The solvent is not particularly limited, a solvent that does not interfere with a reaction of a compound is preferable, and a solvent in which raw materials used in a reaction have high solubility is preferable. Examples thereof include N,N-dimethylformamide (DMF), tetrahydrofuran (THF), and 1,4-dioxane.

In the present embodiment, in the reactions in Process 1 to Process 3, the reaction system may further contain other compounds that do not correspond to the above exemplified compounds in a range in which amide production can be achieved.

In the present embodiment, the reactions in Process 1 to Process 3 may be performed separately or simultaneously, whereby Process 3 according to the present invention is performed simultaneously. In order to more effectively minimize the production of side-reaction products, it is preferable that Process 2 and Process 3 be performed simultaneously.

In the method for producing an amide according to the embodiment described above, the presence and structure of the product can be confirmed by measuring the spectrum obtained by analysis through NMR, IR, mass spectrometry, or the like or elemental analysis or the like. In addition, as necessary, the product may be purified and can be produced by a purification method such as distillation, extraction, recrystallization, and column chromatography.

According to the method for producing an amide of the embodiment, it is possible to produce an amide with very high efficiency. Even the acid anhydride obtained in Process 1 is in a state in which it accepts a nucleophilic species (amine) as an active species. In this method, a cationically active species is additionally formed in Process 2, and the amine is reacted with this for the first time. Since the cationically active species produced here has significantly higher activity than the acid anhydride, the reaction can proceed at a very high rate, and it is possible to significantly minimize the production of byproducts as compared with a conventional method. In addition, even with an amine having low reactivity, which was difficult to react in the conventional method, it is possible to easily cause the reaction to proceed.

### <<Method for producing peptide>>

In the method for producing an amide according to the embodiment of the present invention, when the carboxylic acid is an amino acid or an amino acid derivative and the amine is an amino acid or an amino acid derivative, peptides or proteins can be synthesized. The method for producing an amide includes a method for producing peptides or proteins.

The amide obtained in Process 3 is used as a carboxylic acid in Process 1, after Processes 1 to 3, Processes 1 to 3 are additionally repeated, and a polypeptide chain can be extended.

That is, the carboxylic acid also includes a polypeptide and the amino acid or the amino acid derivative (carboxylic acid) according to the embodiment also includes an amino acid or an amino acid derivative (carboxylic acid) positioned at the C-terminal as a structural unit of the polypeptide. In this manner, the method for producing an amide according to the embodiment is suitable as a method for producing peptides or proteins.

### <<Distribution system reaction device>>

The method for producing an amide according to the embodiment can be performed using a distribution system reaction device. A distribution system reaction device including flow paths for transporting a fluid containing raw materials or an intermediate used in the reaction in the method for producing an amide according to the embodiment and a mixing machine for mixing the fluid may be exemplified. Regarding use of the distribution system reaction device, for example, a reaction with an amine in at least Process 3 may be performed in the distribution system reaction device, reactions of reacting with a base and reacting with an amine in Process 2 and Process 3 may be performed in the distribution system reaction device, and reactions in which, in Processes 1 to 3, carboxylic acids are dehydrated and condensed and then reacted with a base and reacted with an amine may be performed in the distribution system reaction device. Here, the method for producing an amide according to the embodiment is not limited to the method that is performed using the distribution system reaction device. For example, a batch container having a small volume and a high stirring speed may be used. The volume of the mixing part of the batch container may be 1 to 100 mL or 5 to 50 mL.

Hereinafter, a form of a distribution system reaction device according to an embodiment and a method for producing an amide according to an embodiment using the same will be described with reference to Fig. 1.

Fig. 1 is a schematic view showing a schematic configuration of a distribution system reaction device 1. The distribution system reaction device 1 includes a tank 11 in which a first liquid is accommodated, a tank 12 in which a second liquid is accommodated, and a tank 13 in which a third liquid is accommodated.

As an example, the first liquid contains a first carboxylic acid and a second carboxylic acid, the second liquid contains a phosgene or a phosgene equivalent that decomposes in a reaction system and produces a phosgene, and the third liquid contains a base and an amine. As a more specific example, as shown in Fig. 1, the first liquid contains a carboxylic acid and DIEA, the second liquid contains a triphosgene, and the third liquid contains 4-morpholinopyridine and an amine.

Regarding use of the distribution system reaction device, for example, a mixture containing at least the first liquid and the second liquid may be mixed with the third liquid in the distribution system reaction device, and additionally, the first liquid and the second liquid may be mixed in the distribution system reaction device.

The distribution system reaction device 1 includes flow paths f1, f2, f3, f4, and f5 for transporting a fluid. As an example, the inner diameter of the flow path may be 0.1 to 10 mm or 0.3 to 8 mm. The distribution system reaction device 1 includes mixing machines 31 and 32 for mixing fluids. As an example, the inner diameter of the flow path inside the mixing machine may be 0.1 to 10 mm or 0.3 to 8 mm. Examples of mixing machines include a static mixer having no drive unit. A drive unit is a unit that receives power and moves.

The inner diameter of the flow path can be a diameter of the inner portion (a portion through which a fluid passes) of the flow path in the cross section of the flow path in a direction perpendicular to the length direction of the flow path. When the shape of the inner portion of the flow path is not a perfect circle, the inner diameter of the flow path can be a diameter when the shape of the inner portion of the flow path is converted into a perfect circle based on the area.

As an example, the tanks 11, 12, 13, and 14, the mixing machines 31 and 32, and the flow paths f1, f2, f3, f4, and f5 are formed of a resin such as a plastic or an elastomer or a glass material, a metal, a ceramic, or the like.

The tank 11 is connected to a pump 21, and the first liquid accommodated in the tank 11 moves through the flow path f1 due to an operation of the pump 21 and flows into the mixing machine 31. The tank 12 is connected to a pump 22, and the second liquid accommodated in the tank 12 moves through the flow path f2 due to an operation of the pump 22 and flows into the mixing machine 31. Then, the first liquid and the second liquid are mixed by the mixing machine 31 to form a first mixed liquid and the first mixed liquid is sent to the flow path f4. In a procedure after this mixing, carboxylic acids contained in the first liquid are dehydrated and condensed to obtain an acid anhydride (Process 1 in the method for producing an amide). The first mixed liquid containing the obtained acid anhydride flows into the mixing machine 32.

On the other hand, the tank 13 is connected to a pump 23, the liquid accommodated in the tank 13 moves through the flow path f3 due to an operation of the pump 23, flows into the mixing machine 32, and is mixed with the first mixed liquid to form a second mixed liquid, and the second mixed liquid is sent to the flow path f5. In a procedure after this mixing, the acid anhydride obtained in Process 1 reacts with 4-morpholinopyridine contained in the third liquid to form a cationically active species (Process 2 in the method for producing an amide), and subsequently, the obtained cationically active species reacts with an amine contained in the third liquid to obtain an amide (Process 3 in the method for producing an amide). The second mixed liquid containing the produced amide is stored in a tank 14.

According to the distribution system reaction device 1 of the embodiment, it is possible to increase an area for performing heat exchange per volume of the reaction solution. In addition, it is possible to control the reaction time by the flow rate and the length of the flow path. Therefore, it is possible to precisely control the reaction solution, and as a result, it is possible to minimize the progress of undesired side reactions, and it is possible to improve the yield of the desired product.

Since the cationically active species obtained in Process 2 has high activity, it has an advantage that it can also be reacted with an amine having low reactivity, but it is important to control the reaction. In addition, since the acid anhydride obtained in Process 1 has sufficiently high activity, it is important to control the reaction.

According to the distribution system reaction device 1 of the embodiment, when liquids are continuously distributed through the flow paths, an opportunity for compound collision is improved, the reaction can proceed with higher efficiency, and it is easy to minimize side reactions. For example, since the acid anhydride produced in Process 1 can be immediately reacted with 4-morpholinopyridine (base), the time during which the acid anhydride is in an activated state can be shortened, and it is possible to reduce a probability of the occurrence of side reactions such as isomerization.

Here, in the distribution system reaction device according to the present embodiment, the form in which liquids are mixed by a mixing machine has been exemplified. However, since liquids can be mixed simply by communicating the flow paths with each other, the distribution system reaction device of the embodiment does not necessarily include the mixing machine.

As shown here, the method for producing an amide according to the embodiment can be performed by a liquid phase method. For example, a current mainstream method for producing peptides (amides) is a solid phase method, and peptides in a solid phase may be synthesized. On the other hand, the liquid phase method is suitable for large-scale synthesis, and has favorable reactivity because the degree of freedom of molecules is high. The liquid phase method is also effective in reacting with an amine having low reactivity.

Here, in the distribution system reaction device according to the present embodiment, 5 types of compounds to be reacted are separately accommodated in three tanks. However, for example, the compounds may be accommodated in a total of 5 separate tanks and mixed sequentially.

However, as shown as the third liquid of the above embodiment, preferably, 4-morpholinopyridine (base) and an amine are present in the same liquid in advance. That is, Process 2 and Process 3 may be performed simultaneously, and accordingly, it is easy to react immediately the cationically active species having high reactivity produced in Process 2 with a desired amine, the time during which the cationically active species is in an activated state can be shortened, and it is possible to effectively minimize the production of undesired side-reaction products.

### [Examples]

While the present invention will be described below in more detail with reference to examples, the present invention is not limited to the following examples.

### <Example 1> Method for producing amide according to the present invention

### [Raw materials]

Regarding the amino acid used as a carboxylic acid, Fmoc-His(MBom)-OH (commercial product) which is histidine in which the amino group is protected by the Fmoc group and the π position of the histidine side chain is protected by the 4-methoxybenzyloxymethyl (MBom) group was used. Regarding the amino acid used as an amine, H-MePhe-OMe (commercial product) which is phenylalanine in which the carboxylic group is protected by the methyl group and the amino group is methylated was used.

### [Flow synthesis of acid amide]

A coupling reaction between the amino acid used as a carboxylic acid and the amino acid used as an amine was caused. For the coupling reaction, a distribution system reaction device composed of a PTFE tube (an inner diameter of 0.8 mm and an outer diameter of 1.59 mm) and a T-shaped mixer was used. Three unreacted solutions were separately prepared. The first solution was obtained by dissolving Fmoc-His(MBom)-OH used as a carboxylic acid and DIEA in DMF. The second solution was obtained by dissolving triphosgene in THF. The third solution was obtained by dissolving H-MePhe-OMe used as an amine and 4-morpholinopyridine in THF. A ratio of molar concentrations in the distribution system reaction device was 1.0 for H-MePhe-OMe, 0.010 for 4-morpholinopyridine, 0.40 for triphosgene, 3.0 for DIEA, and 2.5 for Fmoc-His(MBom)-OH.

In order to perform coupling in the distribution system reaction device, first, the first solution and the second solution were mixed in a T-shaped mixer, and reacted in the distribution system reaction device for 1 second to obtain a symmetric acid anhydride. Immediately thereafter, a reaction solution containing the symmetric acid anhydride and the third solution were mixed using a new T-shaped mixer, and reacted in the distribution system reaction device for 30 seconds and in a test tube for about 5 minutes after collection. All of these reactions were performed at 30 °C, and 20 seconds was set as a time for heat exchange before the unreacted solutions reached the mixer. Various solutions were discharged using a syringe pump. The flow rate of each pump was 2.0 mL/min for the first solution, 1.2 mL/min for the second solution, and 2.0 mL/min for the third solution.

The reaction in Process 1 in the method for producing an amide in Example 1 is shown below. 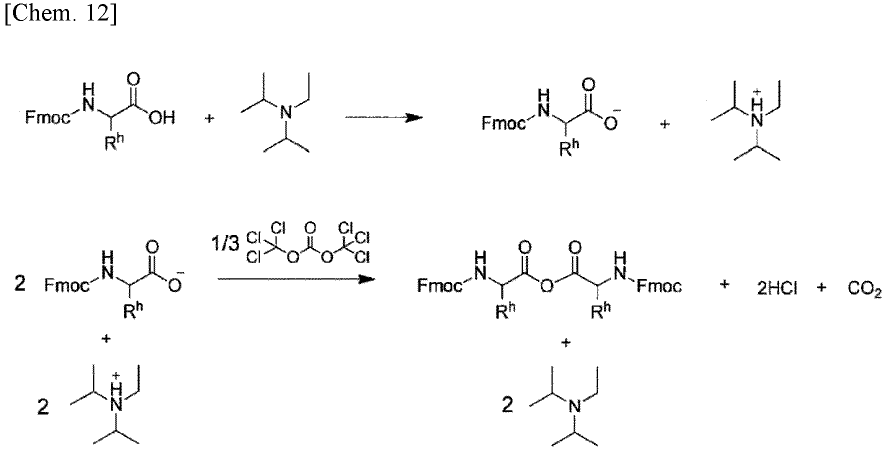 [in the formula, R^{h} represents a histidine side chain (protected by the protecting group MBom in the present example)]

The reaction in Process 2 in the method for producing an amide in Example 1 is shown below. 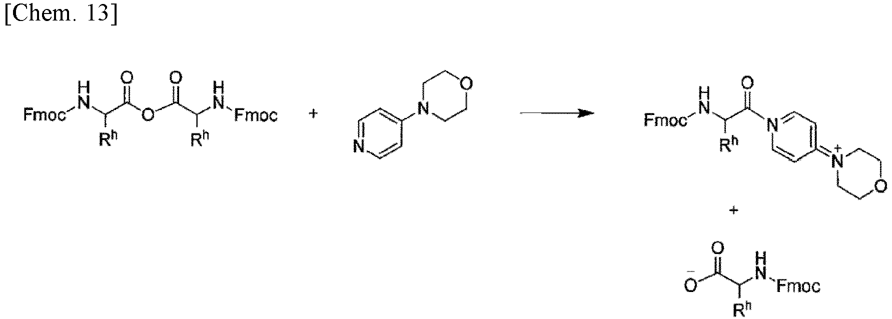 [in the formula, R^{h} represents a histidine side chain (protected by the protecting group MBom in the present example)]

The reaction in Process 3 in the method for producing an amide in Example 1 is shown below. 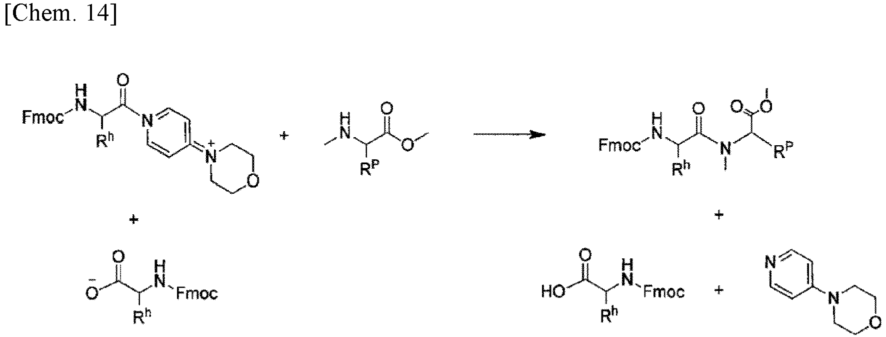 [in the formula, R^{h} represents a histidine side chain (protected by the protecting group MBom in the present example), and R^{p} represents a phenylalanine side chain]

### [Analysis method]

The desired product was isolated using GPC and identification was performed through H¹-NMR at 400 MHz.

Analysis of the isomerization rate was performed using GC-MS.

The sample was prepared as follows. After protecting groups of the obtained dipeptide were removed, the peptide/amino acid derivatives were hydrolyzed in deuterium hydrochloric acid, the sample was esterified with deuteride in methyl alcohol, a reagent was evaporated, and the residue was then acylated using trifluoroacetic anhydride or pentafluoropropionic anhydride.

The yield of the desired product was calculated from the weight of the isolated and purified desired product. That is, the molar equivalent ratio of the amine was set to 1.0, and a ratio of amine coupling was calculated from the weight of the isolated dipeptide.

### [Results]

NMR data of the obtained dipeptide is shown below.
¹H NMR (400 MHz, CDCl₃, major rotamer):6 7.78-6.85 (m, 20 H), 5.33-5.22 (m, 3 H), 4.79-4.74 (m, 1 H), 4.44-4.30 (m, 4 H), 4.15 (t, J = 6.8 Hz, 1 H), 3.79 (s, 3 H),3.72 (s, 3 H), 3.33 (dd, J = 5.5, 14.5 Hz, 1 H), 3.03 (dd, J = 6.8, 14.5 Hz, 1 H),2.95-2.85 (m, 2 H), 2.67 (s, 3 H).

Analyzing the product after the reaction showed that the dipeptide that was the desired product had a coupling yield of 84%, of which an isomerization ratio of the His site was 1.1%. In addition, no side-reaction products other than isomerization were detected.

According to the method in Example 1, although the molar concentration ratio of the carboxylic acid to the amine was 1 :2.5, a high coupling yield of 80% or more could be achieved in a short time of 5 minutes. In addition, the generation rate of the epimer contained in the desired product was about 1% and no other byproducts were detected.

### <Comparative Example 1> Mixed acid anhydride method

### [Raw materials]

Regarding the amino acid used as a carboxylic acid, Fmoc-His(MBom)-OH which is histidine in which the amino group is protected by the Fmoc group and the π position of the side chain is protected by the 4-methoxybenzyloxymethyl (MBom) group was used. Regarding the amino acid used as an amine, H-MePhe-OMe which is phenylalanine in which the carboxylic group is protected by the methyl group and the amino group is methylated was used.

### [Flow synthesis of acid amide]

A coupling reaction between the amino acid used as a carboxylic acid and the amino acid used as an amine was caused. For the coupling reaction, a distribution system reaction device composed of a PTFE tube (an inner diameter of 0.8 mm and an outer diameter of 1.59 mm) and a T-shaped mixer was used. Three unreacted solutions were separately prepared. The first solution was obtained by dissolving Fmoc-His(MBom)-OH used as a carboxylic acid, N-methylmorpholine, and DIEA in DMF. The second solution was obtained by dissolving isopropyl chloroformate in THF. The third solution was obtained by dissolving H-MePhe-OMe used as an amine and 4-morpholinopyridine in THF. A ratio of molar concentrations in the distribution system reaction device was 1.0 for H-MePhe-OMe, 0.010 for 4-morpholinopyridine, and 1.0 for the remaining Fmoc-His(MBom)-OH, N-methylmorpholine, DIEA, and isopropyl chloroformate.

In order to perform coupling in the distribution system reaction device, first, the first solution and the second solution were mixed in a T-shaped mixer and reacted in the distribution system reaction device for 20 seconds to obtain a mixed acid anhydride. Immediately thereafter, a reaction solution containing the mixed acid anhydride and the third solution were mixed using a new T-shaped mixer and reacted in the distribution system reaction device for 30 seconds and in a test tube for about 5 minutes after collection. All of these reactions were performed at 30 °C and 20 seconds was set as a time for heat exchange before the unreacted solutions reached the mixer. Various solutions were discharged using a syringe pump. The flow rate of each pump was 1.2 mL/min for the first solution, 2.0 mL/min for the second solution, and 2.0 mL/min for the third solution.

### [Analysis method]

Analysis was performed in the same method as in Example 1.

Here, in isolation of the ester and the desired product, since the desired product and the ester had the same polarity, the ester and the desired product were isolated using GPC, and identification was performed through H¹-NMR at 400 MHz. The yields of the ester and the desired product were calculated from the ratio of the isolation yield of the mixture to the peak area of the ester and the desired product obtained through NMR.

### [Results]

Analyzing the product after the reaction showed that the dipeptide that was the desired product had a coupling yield of 26.4%. 33.5% of the raw material carboxylic acid was consumed by esterification which was a side reaction.

The acylpyridinium species (cationically active species) which was an activated carboxylic acid should be subjected to a nucleophilic attack from an amine in order to obtain an intended product. However, in the method of Comparative Example 1, it was thought that the acylpyridinium species (cationically active species) was subjected to a nucleophilic attack by its counter anion at a rate equal to or higher than that of the amine. It is thought that, in the ester which was a side-reaction product obtained in the reaction with counter anions, 33.5% of the carboxylic acid was consumed during the same reaction, and as a result, the yield of the dipeptide that was the desired product was lowered to 26.4%.

### [Reference Signs List]

- 1: Distribution system reaction device
- 11, 12, 13, 14: Tank
- 21, 22, 23: Pump
- 31, 32: Mixing machine
- f1, f2, f3, f4, f5: Flow path

## Claims

1. A method for producing an amide, the method comprising:
dehydrating and condensing carboxylic acids to obtain an acid anhydride; and
reacting the acid anhydride with a base to obtain an acylpyridinium cation,
which is then reacted with an amine simultaneously;
wherein the base is any one or more selected from the group consisting of pyridine and pyridine derivatives,
wherein the carboxylic acids are amino acids or amino acid derivatives, and wherein the amine is an amino acid or an amino acid derivative.

2. The method for producing an amide according to claim 1, wherein a phosgene or a phosgene equivalent that decomposes in a reaction system and produces a phosgene is reacted and the carboxylic acids are dehydrated and condensed.

3. The method for producing an amide according to claim 1 or 2, wherein carboxylic acids of the same type are dehydrated and condensed.

4. The method for producing an amide according to any one of claims 1 to 3,
wherein the base is any one or more selected from the group consisting of 4-morpholinopyridine, N,N-dimethyl-4-aminopyridine, 4-pyrrolidinopyridine, pyridine and 4-methoxypyridine.

5. The method for producing an amide according to any one of claims 1 to 4,
wherein the nucleophilicity of the amine is lower than the nucleophilicity of 18 amino acids excluding valine and isoleucine from 20 amino acids which constitute proteins and are encoded as genetic information.

6. The method for producing an amide according to claim 5, wherein the amine is valine, isoleucine, an N-alkylated amino acid, or derivatives thereof.

7. The method for producing an amide according to any one of claims 1 to 6,
wherein the reaction with the amine is performed in a distribution system reaction device (1).

8. The method for producing an amide according to claim 7, wherein the reaction with the base is additionally performed in the distribution system reaction device (1) after the carboxylic acids are dehydrated and condensed.

## Patentansprüche

1. Verfahren zur Herstellung eines Amids, wobei das Verfahren umfasst:
Dehydratisieren und Kondensieren von Carbonsäuren, um ein Säureanhydrid zu erhalten; und
Umsetzen des Säureanhydrids mit einer Base, um ein Acylpyridiniumkation zu erhalten, das dann gleichzeitig mit einem Amin umgesetzt wird;
wobei die Base eine oder mehrere ist, ausgewählt aus der aus Pyridin und Pyridinderivaten bestehenden Gruppe,
wobei die Carbonsäuren Aminosäuren oder Aminosäurederivate sind und
wobei das Amin eine Aminosäure oder ein Aminosäurederivat ist.

2. Verfahren zur Herstellung eines Amids nach Anspruch 1, wobei ein Phosgen oder ein Phosgenäquivalent, das sich in einem Reaktionssystem zersetzt und ein Phosgen erzeugt, umgesetzt wird und die Carbonsäuren dehydratisiert und kondensiert werden.

3. Verfahren zur Herstellung eines Amids nach Anspruch 1 oder 2, wobei gleichartige Carbonsäuren dehydratisiert und kondensiert werden.

4. Verfahren zur Herstellung eines Amids nach einem der Ansprüche 1 bis 3, wobei die Base eine oder mehrere ist, ausgewählt aus der Gruppe bestehend aus 4-Morpholinopyridin, N,N-Dimethyl-4-aminopyridin, 4-Pyrrolidinopyridin, Pyridin und 4-Methoxypyridin.

5. Verfahren zur Herstellung eines Amids nach einem der Ansprüche 1 bis 4, wobei die Nukleophilie des Amins geringer ist als die Nukleophilie von 18 Aminosäuren, ausgenommen Valin und Isoleucin, aus 20 Aminosäuren, die Proteine bilden und als genetische Information kodiert sind.

6. Verfahren zur Herstellung eines Amids nach Anspruch 5, wobei das Amin Valin, Isoleucin, eine N-alkylierte Aminosäure oder Derivate davon ist.

7. Verfahren zur Herstellung eines Amids nach einem der Ansprüche 1 bis 6, wobei die Umsetzung mit dem Amin in einer Verteilersystem-Reaktionsvorrichtung (1) durchgeführt wird.

8. Verfahren zur Herstellung eines Amids nach Anspruch 7, wobei die Reaktion mit der Base zusätzlich in der Verteilersystem-Reaktionsvorrichtung (1) durchgeführt wird, nachdem die Carbonsäuren dehydratisiert und kondensiert wurden.

## Revendications

1. Procédé de production d'un amide, le procédé comprenant les étapes consistant à : déshydrater et condenser des acides carboxyliques pour obtenir un anhydride acide ; et faire réagir l'anhydride acide avec une base pour obtenir un cation acylpyridinium, qui est ensuite mis en réaction avec une amine de manière simultanée ;
dans lequel la base est l'un ou plusieurs quelconques sélectionnés à partir du groupe consistant en la pyridine et les dérivés de pyridine,
dans lequel les acides carboxyliques sont des acides aminés ou des dérivés d'acide aminé, et
dans lequel l'amine est un acide aminé ou un dérivé d'acide aminé.

2. Procédé de production d'un amide selon la revendication 1, dans lequel un phosgène ou un équivalent de phosgène qui se décompose dans un système de réaction et produit un phosgène est mis en réaction et les acides carboxyliques sont déshydratés et condensés.

3. Procédé de production d'un amide selon la revendication 1 ou 2, dans lequel des acides carboxyliques du même type sont déshydratés et condensés.

4. Procédé de production d'un amide selon l'une quelconque des revendications 1 à 3, dans lequel la base est l'une ou plusieurs quelconques sélectionnées à partir du groupe consistant en la 4-morpholinopyridine, la N,N-diméthyl-4-aminopyridine, la 4-pyrrolidinopyridine, la pyridine et la 4-méthoxypyridine.

5. Procédé de production d'un amide selon l'une quelconque des revendications 1 à 4, dans lequel la nucléophilie de l'amine est inférieure à la nucléophilie de 18 acides aminés, à l'exclusion de la valine et de l'isoleucine, parmi 20 acides aminés qui constituent des protéines et sont codés en tant qu'informations génétiques.

6. Procédé de production d'un amide selon la revendication 5, dans lequel l'amine est la valine, l'isoleucine, un acide aminé N-alkylé ou des dérivés de ceux-ci.

7. Procédé de production d'un amide selon l'une quelconque des revendications 1 à 6, dans lequel la réaction avec l'amine est effectuée dans un dispositif de réaction de système de distribution (1).

8. Procédé de production d'un amide selon la revendication 7, dans lequel la réaction avec la base est en outre effectuée dans le dispositif de réaction de système de distribution (1) après que les acides carboxyliques ont été déshydratés et condensés.
